# EUROPÄISCHE PATENTSCHRIFT

(11) **EP 0 647 215 B1**
(45) Veröffentlichungstag und Bekanntmachung des Hinweises auf die Patenterteilung: **10.09.1997**
(21) Anmeldenummer: 93912890.6
(22) Anmeldetag: 04.06.1993
(51) Int. Cl.: C07C 41/09, C07C 43/13

(54) **VERFAHREN ZUR HERSTELLUNG VON OLIGOGLYCERINGEMISCHEN MIT ERHÖHTEM DIGLYCERINGEHALT**
PROCESS FOR PRODUCING OLIGOCLYCERINE MIXTURES WITH INCREASED DIGLYCERINE CONTENT
PROCEDE DE FABRICATION DE MELANGES D'OLIGOGLYCERINE A TENEUR ELEVEE EN DIGLYCERINE

(30) Priorität: 12.06.1992 US 898061
(43) Veröffentlichungstag der Anmeldung: 12.04.1995
(73) Patentinhaber: Henkel Kommanditgesellschaft auf Aktien, 40191 Düsseldorf (DE)
(72) Erfinder: HEES, Udo, D-5440 Mayen 1 (DE); BUNTE, Reinhard, D-4047 Dormagen 11 (DE); HACHGENEI, Johannes, W., D-4000 Düsseldorf 13 (DE); KUHM, Peter, D-4010 Hilden (DE); HARRIS, Eugene, G., West Chester, OH 45069 (US)
(86) Internationale Anmeldenummer: EP9301418
(87) Internationale Veröffentlichungsnummer: WO9325511

(56) Entgegenhaltungen:
- WO-A-92/05133
- DATABASE WPI Week 8208, Derwent Publications Ltd., London, GB; AN 82-14626E; & JP-A-57 007 432

## Beschreibung

Die Erfindung betrifft ein Verfahren zur Herstellung von Oligoglyceringemischen mit erhöhtem Gehalt an Diglycerin, bei dem man Glycerin in Gegenwart von Zeolithen bei erhöhter Temperatur kondensiert.

### Stand der Technik

Diglycerin hat als Ausgangsmaterial für die Herstellung von Fettsäureestern große Bedeutung erlangt. Derartige Ester finden Verwendung als Emulgatoren in der Nahrungsmittelindustrie und der Kosmetik sowie in verschiedenen technischen Anwendungen, beispielsweise als Schmierstoffe oder Stabilisatoren für PVC **[J.Am.Oil. Chem.Soc. 66, 153 (1989)].**

Zur Herstellung von Diglycerin wird in der Regel von Glycerin ausgegangen, das mit Glycidol **[Fette,Seifen,Anstrichmitt., 88 101 (1986)]** oder Epichlorhydrin **[EP 0 333 984 A1]** umgesetzt wird. Die Reaktion verläuft jedoch wenig selektiv, zudem sind Glycidol und Epichlorhydrin schwer zu handhaben und stellen hohe Anforderungen an den Arbeitsschutz.

Alternativ hierzu kann Glycerin in Gegenwart von Alkalibasen kondensiert werden. Auf diesem Wege sind jedoch nur Gemische erhältlich, die neben Diglycerin noch höhere Homologe sowie nicht zu vernachlässigende Mengen an nichtumgesetztem Glycerin enthalten. Das Diglycerin muß aus diesen Gemischen destillativ abgetrennt werden, was mit einem hohem Aufwand an Zeit und Energie verbunden ist. Aus diesem Grunde wird die Herstellung von Oligoglyceringemischen angestrebt, die einen möglichst hohen Diglycerinanteil aufweisen. Aufgrund der hohen Produktionsmengen stellt dabei die Erhöhung des Diglycerinanteils um 1 Gew.-% bereits einen ökonomisch bedeutsamen Fortschritt dar.

Durch das Verfahren gemäß WO 92/05133, lassen sich, Oligoglyceringemische mit hohem Diglyceringehalt dadurch herstellen, daß man Glycerin in Gegenwart von Silicatverbindungen kondensiert, das Kondensationswasser kontinuierlich aus der Reaktionsmischung entfernt und die Reaktion abbricht, sobald die zur Bildung von Diglycerin theoretisch erforderliche Menge Wasser abgeschieden worden ist.

Die Aufgabe der Erfindung bestand somit darin, ein verbessertes Verfahren zur Herstellung von Oligoglyceringemischen mit erhöhtem Diglyceringehalt zu entwickeln.

### Beschreibung der Erfindung

Gegenstand der Erfindung ist ein Verfahren zur Herstellung von Oligoglyceringemischen mit erhöhtem Diglyceringehalt, das sich dadurch gekennzeichnet, daß man Glycerin in Gegenwart von Zeolithen der Formel **(I)**,

**M**_{**2/z**}**O ∗ Al**_{**2**}**O**_{**3**} **∗ x SiO**_{**2**} **∗ y H**_{**2**}**O (I)**

in der M für ein Alkali- oder Erdalkalimetall der Wertigkeit z, x für Zahlen von 1,8 bis 12 und y für Zahlen von 0 bis 8 steht, kondensiert, das Kondensationswasser kontinuierlich aus der Reaktionsmischung entfernt und die Reaktion abbricht, sobald die zur Bildung von Diglycerin theoretisch erforderliche Menge Wasser abgeschieden worden ist.

Überraschenderweise wurde gefunden, daß Zeolithe nicht nur die Eigenkondensation des Glycerins katalysieren, sonden auch die Bildung solcher Oligoglyceringemische begünstigen, die einen gegenüber dem Stand der Technik erhöhten Diglycerin- sowie einen verminderten Ascheanteil aufweisen. Die Katalysatoren sind in der Reaktionsmischung unlöslich und können nach Beendigung der Reaktion problemlos abgetrennt werden, so daß feststoffarme Oligoglyceringemische erhalten werden.

Unter **Zeolithen** sind im Sinne des erfindungsgemäßen Verfahrens gegebenenfalls wasserhaltige Alkali- oder Erdalkali-Alumosilicate der allgemeinen Formel (I) zu verstehen, die natürlicher oder synthetischer Herkunft sein können. Typisch Beispiele sind die natürlich vorkommenden Mineralien Clinoptilolith, Erionit oder Chabasit. Bevorzugt sind jedoch synthetische Zeolithe, beispielsweise

Zeolith X Na₈₆[(AlO₂)₈₆(SiO₂)₁₀₆] ∗ 264 H₂O

Zeolith Y Na₅₆[(AlO₂)₅₆(SiO₂)₁₃₆] ∗ 325 H₂O

Zeolith L K₉[(AlO₂)₉(SiO₂)₂₇] ∗ 22 H₂O

Mordenit Na_{8,7}[(AlO₂)_{8,7}(SiO₂)_{39,3}] ∗ 24 H₂O

und insbesondere

Zeolith A Na₁₂[(AlO₂)₁₂(SiO₂)₁₂] ∗ 27 H₂O.

Die Zeolithe können in der Kondensation in Mengen von 1 bis 10, vorzugsweise 2 bis 5 Gew.-% - bezogen auf das Glycerin - eingesetzt werden. Die Temperatur, bei der die Kondensation des Glycerins durchgeführt wird, kann 200 bis 260, vorzugsweise 240 bis 250°C betragen.

Zur Durchführung der Kondensationsreaktion werden Glycerin und Zeolithe vorgelegt und in einer Inertgasatmosphäre auf die Reaktionstemperatur erhitzt. Zur Verlagerung des Gleichgewichtes wird das entstehende Kondensationswasser beispielsweise über einen Wasserabscheider abgetrennt. Die Reaktion wird abgebrochen, wenn die zur Bildung von Diglycerin theoretisch erforderliche Menge Wasser abgeschieden worden ist. Im allgemeinen beträgt die Reaktionszeit 1 bis 30, vorzugsweise 3 bis 25 h. Im Anschluß wird der Katalysator aus dem Reaktionsgemisch, beispielsweise durch Druckfiltration abgetrennt.

Die Abtrennung des Diglycerins aus dem gebildeten Oligoglyceringemisch kann beispielsweise durch Destillation im Hochvakuum erfolgen. Für eine Vielzahl von Anwendungen können jedoch die erfindungsgemäßen Oligoglyceringemische ohne weitere Auftrennung eingesetzt werden.

### Gewerbliche Anwendbarkeit

Die nach dem erfindungsgemäßen Verfahren erhältlichen Oligoglyceringemische mit erhöhtem Diglyceringehalt eignen sich zur Herstellung von Chemieprodukten, insbesondere Fettsäureestern sowie als Emulgatoren in Nahrungsmitteln und kosmetischen Produkten, in denen sie in Mengen von 0,1 bis 25, vorzugsweise 1 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

In einem 1-l-Dreihalskolben mit Rückflußkühler und Wasserabscheider wurden 552,0 g (6 Mol) Glycerin und 13,3 g Zeolith Na-A (Wessalith^{(R)} CD, Fa.Degussa AG, Frankfurt, FRG) - entsprechend 2,4 Gew.-% bezogen auf das Glycerin - vorgelegt und unter Stickstoffabdeckung über einen Zeitraum von 22 h auf 240°C erhitzt. Anschließend wurde das Reaktionsprodukt abgekühlt und über eine Drucknutsche (Betriebsdruck : 3 bar) abfiltriert. Das als Filtrat anfallende Oligoglyceringemisch zeigte die folgende Zusammensetzung:
15,4 Gew.-% Glycerin
32,3 Gew.-% Diglycerin
20,5 Gew.-% Triglycerin
31,8 Gew.-% höhere Oligoglyceringemische
Aschegehalt (Veraschung über 2 h bei 800°C) : 0,5 Gew.-%

### Beispiel 2:

Beispiel 1 wurde unter Einsatz von 552 g Glycerin und 13,3 g Zeolith NaX-Granulat (Zeolite 13X, Fa.Union Carbide, USA) wiederholt. Das nach Abtrennung des im Reaktionsgemisch unlöslichen Katalysators anfallende Oligoglyceringemisch zeigte die folgende Zusammensetzung:
9,5 Gew.-% Glycerin
27,6 Gew.-% Diglycerin
20,0 Gew.-% Triglycerin
42,9 Gew.-% höhere Oligoglyceringemische
Aschegehalt (Veraschung über 2 h bei 800°C) : 0,6 Gew.-%

### Beispiel 3

Beispiel 1 wurde unter Einsatz von 552 g Glycerin und 13,3 g Zeolith NaX-Pulver (Zeolite 13X, Fa.Union Carbide, USA) wiederholt. Das nach Abtrennung des im Reaktionsgemisch unlöslichen Katalysators anfallende Oligoglyceringemisch zeigte die folgende Zusammensetzung:
9,6 Gew.-% Glycerin
30,9 Gew.-% Diglycerin
22,0 Gew.-% Triglycerin
37,5 Gew.-% höhere Oligoglyceringemische
Aschegehalt (Veraschung über 2 h bei 800°C) : 1,2 Gew.-%

### Vergleichsbeispiel V1:

Beispiel 1 wurde unter Einsatz von 552 g Glycerin und 13,3 g Natriumsilicat (NaSKS6, Fa.Hoechst AG, Frankfurt, FRG) wiederholt. Der Katalysator löste sich im Glycerin auf und konnte nach der Reaktion nicht wieder abgetrennt werden. Das resultierende Oligoglyceringemisch zeigte die folgende Zusammensetzung:
8,5 Gew.-% Glycerin
29,7 Gew.-% Diglycerin
23,0 Gew.-% Triglycerin
38,8 Gew.-% höhere Oligoglyceringemische
Aschegehalt (Veraschung über 2 h bei 800°C) : 2,6 Gew.-%

## Patentansprüche

1. Verfahren zur Herstellung von Oligoglyceringemischen mit erhöhtem Diglyceringehalt, **dadurch gekennzeichnet,** daß man Glycerin in Gegenwart von Zeolithen der Formel **(I)**,
**M**_{**2/z**}**O ∗ Al**_{**2**}**O**_{**3**} **∗ x SiO**_{**2**} **∗ y H**_{**2**}**O (I)**
in der M für ein Alkali- oder Erdalkalimetall der Wertigkeit z, x für Zahlen von 1,8 bis 12 und y für Zahlen von 0 bis 8 steht, kondensiert, das Kondensationswasser kontinuierlich aus der Reaktionsmischung entfernt und die Reaktion abbricht, sobald die zur Bildung von Diglycerin theoretisch erforderliche Menge Wasser abgeschieden worden ist.

2. Verfahren nach Anspruch 1, **dadurch gekennzeichnet,** daß man Zeolithe ausgewählt der Gruppe, die von Zeolith X, Zeolith Y, Zeolith L, Zeolith A und Mordenit gebildet wird, einsetzt.

3. Verfahren nach den Ansprüchen 1 und 2, **dadurch gekennzeichnet,** daß man die Zeolithe in Mengen von 1 bis 10 Gew.-% - bezogen auf das Glycerin - einsetzt.

4. Verfahren nach mindestens einem der Ansprüche 1 bis 3 **dadurch gekennzeichnet,** daß man die Kondensation bei Temperaturen von 200 bis 260°C durchführt.

5. Verfahren nach mindestens einem der Ansprüche 1 bis 4, **dadurch gekennzeichnet**, daß man die Zeolithe nach der Reaktion aus dem Oligoglyceringemisch abtrennt.

## Claims

1. A process for the production of oligoglycerol mixtures of increased diglycerol content, **characterized in that** glycerol is condensed in the presence of zeolites corresponding to formula **(I)**
**M**_{**2/z**}**O ∗ Al**_{**2**}**O**_{**3**} **∗ x SiO**_{**2**} **∗ y H**_{**2**}**O (I)**
in which M is an alkali metal or alkaline earth metal having a valency of z, x is a number of 1.8 to 12 and y is a number of 0 to 8,
the water of condensation is continuously removed from the reaction mixture and the reaction is terminated when the quantity of water theoretically necessary for the formation of diglycerol has been separated.

2. A process as claimed in claim 1, **characterized in that** zeolites selected from the group consisting of zeolite X, zeolite Y, zeolite L, zeolite A and mordenite are used.

3. A process as claimed in claims 1 and 2, **characterized in that** the zeolites are used in quantities of 1 to 10% by weight, based on the glycerol.

4. A process as claimed in at least one of claims 1 to 3, **characterized in that** the condensation is carried out at temperatures of 200 to 260°C.

5. A process as claimed in at last one of claims .1 to 4, **characterized in that** the zeolites are separated from the oligoglycerol mixture after the reaction.

## Revendications

1. procédé de fabrication de mélanges d'oligoglycérines à à teneur accrue en diglycérine,
caractérisé en ce qu'
on condense de la glycérine en présence de zéolites de formule (I)
M_{2/z}O ∗ Al₂O₃ ∗ x SiO₂ ∗ y H₂O (I)
où M est un métal alcalin ou alcalino-terreux de valence z, x représente des nombres de 1,8 à 12 et y des nombres de 0 à 8, on élimine en continu l'eau de la condensation du mélange réactionnel et on interrompt la réaction, dès qu'on a séparé la quantité d'eau théoriquement nécessaire à la formation de diglycérine.

2. Procédé selon la revendication 1,
caractérisé en ce qu'
on utilise des zéolites choisies dans le groupe formé par les zéolite X, zéolite Y, zéolite L, zéolite A et Mordénite.

3. Procédé selon les revendications 1 et 2,
caractérisé en ce qu'
on utilise les zéolites en des quantités de 1 à 10 % en poids, par rapport à la glycérine.

4. Procédé selon au moins l'une des revendications 1 à 3,
caractérisé en ce qu'
on réalise la condensation à des températures de 200 à 260°C.

5. Procédé selon au moins l'une des revendications 1 à 4,
caractérisé en ce qu'
on sépare les zéolites du mélange des oligoglycérines après la réaction.
